# EUROPEAN PATENT SPECIFICATION

(11) **EP 3 244 906 B1**
(45) Date of publication and mention of the grant of the patent: **19.02.2020**
(21) Application number: 16702273.0
(22) Date of filing: 13.01.2016
(51) Int. Cl.: A61K 36/9068, A61K 9/48

(54) **VEGETARIAN CAPSULES CONTAINING SUPERCRITICAL HERBAL EXTRACTS**
VEGETARISCHE KAPSELN MIT SUPERKRITISCHEN KRÄUTEREXTRAKTEN
CAPSULES VÉGÉTARIENNES CONTENANT DES EXTRAITS D'HERBES SUPERCRITIQUES

(30) Priority: 13.01.2015 US 201562102609 P
(43) Date of publication of application: 22.11.2017
(73) Proprietor: New Chapter, Inc., Brattleboro, VT 05301 (US)
(72) Inventor: PINKNEY, Sheila, Momaney, Brattleboro, Vermont 05301 (US); CAMMARN, Stephen, Richard, Cincinnati, Ohio 45202 (US); SIMS, Joshua, Ralph, Cincinnati, Ohio 45202 (US)
(74) Representative: P&G Patent Germany
(86) International application number: PCT/US2016/013239
(87) International publication number: WO 2016/115244

(56) References cited:
- US-A1- 2004 062 778
- US-A1- 2005 123 630
- US-B1- 6 261 607
- US-B1- 6 387 416
- Anonymous: "Ginger Force(TM) is a full-spectrum ginger root extract from New Chapter", New Chapter , 7 July 2015 (2015-07-07), page 12pp, XP055254479, Retrieved from the Internet: URL:https://web.archive.org/web/2015070712 5939/http://www.newchapter.com/force-of-th e-whole/ginger-force [retrieved on 2016-03-02]
- Anonymous: "MITOCHONDRIAL ENERGY OPTIMIZER WITH BioPQQ, 120 Caps", Life extension vitamins , 23 December 2014 (2014-12-23), XP055254483, Retrieved from the Internet: URL:https://web.archive.org/web/2014122302 1354/http://www.lifeextensionvitamins.com/ mienopwibili.html [retrieved on 2016-03-02]
- Anonymous: "Ginger Force", New Chapter , 10 January 2015 (2015-01-10), XP055254496, Retrieved from the Internet: URL:https://web.archive.org/web/2015011013 3850/http://www.newchapter.com/force-of-th e-whole/ginger-force#supplement-facts [retrieved on 2016-03-02]
- DATABASE GNPD [Online] MINTEL; November 2014 (2014-11), anonymous: "liquid capsules", XP002755004, retrieved from gnpd Database accession no. 2783063
- DATABASE GNPD [Online] MINTEL; May 2011 (2011-05), anonymous: "Chlorophyll Liquid Veggie Capsules", XP002755005, retrieved from Mintel Database accession no. 1541234

## Description

### FIELD OF THE INVENTION

This invention relates generally to capsules containing dietary supplements, in particular supercritical herbal extracts contained in vegetarian capsule shells that meet the non-GMO Project Standards.

### BACKGROUND OF THE INVENTION

Practicing good nutrition is challenging. Some people seek supplements to provide additional nutrients and improve their health and wellness. Many consumers seek herbal supplements that provide more of the desired herb than generally found in a user's diet.

Consumers also want a convenient way to take their supplements. Many consumers find taking several capsules at once, or over the course of the day, inconvenient and also find this regimen difficult to start and maintain. However, if the capsules are too large, consumers can have difficulty swallowing the capsules. Thus, it can be advantageous to use supercritical extracts (SCE), including SCEs of herbs, which can deliver a highly concentrated form of the supplement.

Some consumers want a vegetarian capsule that is also compliant with the non-GMO Project Standard. However, some SCEs can interact with and damage capsule shells, especially vegetarian shells. Thus, many capsule shells containing SCEs are not vegetarian and/or are not GMO-free.

Thus, there remains a need for a stable non-GMO capsule that contains a supplement, particularly a supercritical extract of an herbal, where the capsule shell is vegetarian.

### SUMMARY OF THE INVENTION

According to the present invention a capsule is provided, the capsule comprising:
a. a shell containg gellan gum and HPMC;
b. a fill phase comprising beeswax, candelilla wax, and a supercritical extract of an herb;
wherein the capsule is vegetarian and meets the non-GMO Project Standards;
wherein the capsule comprises greater than 0% and less than 5%, by weight of the capsule, beeswax; and
wherein at least 98% of the capsules pass the Cracking Test,
wherein the supercritical extract of an herb comprises a supercritical extract of ginger and the supercritical extract of ginger is less than 10% of the fill phase, and wherein the capsule comprises a volume greater than 0.61 mL.

### DETAILED DESCRIPTION OF THE INVENTION

Many consumers want to improve their health and wellness by taking capsules containing herbal supplements. They want the herbal supplement to be convenient to administer and thus it may be advantageous to use highly concentrated forms of the herbal extract, including SCEs. Consumers may also desire the capsule to be vegetarian and meet the non-GMO Project Standard.

However, when some SCEs are put in vegetarian, non-GMO capsule shells, the shell appeared cracked, broken, split, crazed or otherwise compromised. Typically this occurs outside the sealing zone, often in the cap. Surprisingly, during testing only certain capsules containing specific SCEs were cracked. In some instances, the crack was large enough to cause the fill phase to leak from the capsule and even coat the other capsules. This can make it more difficult to put the capsules in a package because the packaging equipment can become dirty with build-up from the leaking fill, which hinders the operating capability of the equipment. Furthermore, a leaking broken capsule can be undesirable to consumers because it is messy and can also negatively impact stability during shelf life.

It was found, that in order to have a vegetarian capsule that meets the non-GMO Project Standards for some supercritical herbal extracts, it can be necessary to make certain modifications, in order to prevent cracked capsule shells. According to the present invention a capsule is provided, the capsule comprising:
a. a shell containg gellan gum and HPMC;
b. a fill phase comprising beeswax, candelilla wax, and a supercritical extract of an herb;
wherein the capsule is vegetarian and meets the non-GMO Project Standards;
wherein the capsule comprises greater than 0% and less than 5%, by weight of the capsule, beeswax; and
wherein at least 98% of the capsules pass the Cracking Test,
wherein the supercritical extract of an herb comprises a supercritical extract of ginger and the supercritical extract of ginger is less than 10% of the fill phase, and wherein the capsule comprises a volume greater than 0.61 mL.

As used herein, the term "cracking" refers to breaking, splitting, crazing, and the like. Cracking can generally occur outside the capsule's sealing zone and can sometimes be in the cap.

As used herein, the term "non-GMO" means that the product has been verified as fully compliant to the Non-GMO Project Standard. Meets the standards of the non-GMO Project Standard as of May 21, 2014, and thus the product can be labeled Non-GMO Project Verified.

As used herein, the term "supplement" refers to a composition intended to supplement a diet of food and water, where the diet is sufficient to support life. A supplement may contain vitamins, minerals, herbs or other botanicals, amino acids, enzymes, organ tissues, glandular metabolites, or combinations thereof. A supplement may be an extract or concentrate of a particular food source or a particular nutrient. Supplements may be administered by any convenient means, including parenteral or enteral routes. Enteral routes may include oral, gastric, or subgastric administration, including rectal administration. In one example, the supplements of the present invention are administered orally.

As used herein, the term "supercritical fluid" refers to a gas that is heated to a temperature critical point, over which the gas will maintain its gaseous state and not turn to a liquid regardless of pressure. A gas heated to a temperature above its critical point will become very dense on compression, so that its characteristics resemble those of a liquid, but will not become liquid. Carbon dioxide is commonly used in applications requiring a supercritical fluid. The general properties of supercritical fluids and the general use of supercritical fluids in extraction processes are described in, e.g. Taylor, Supercritical Fluid Extraction, Wiley, 1996; McHugh and Krukonis, Supercritical Fluid Extraction: Principles and Practice, 2nd ed., Butterworth-Heinemann, 1994; and Williams and Clifford, Supercritical Fluid Methods and Protocols, Humana Press, 2000.

As used herein, the term "supercritical extraction" refers to the technique in which hydrophobic compounds can be extracted from samples utilizing a supercritical fluid. The solvation power of a supercritical fluid is increased as the pressure and temperature are increased above their critical points, producing an effective solvent for the isolation of hydrophobic molecules.

As used herein, the terms "hydroalcoholic extraction" or "hydroethanolic extraction" refer to the technique in which hydrophilic compounds can be extracted from a sample utilizing a solution of alcohol and water, followed by evaporation of the solution to produce an extract consisting of dissolved solids. In the case of hydroethanolic extraction, the alcohol is ethanol.

As used herein, the term "mammal" refers to any vertebrate of the class Mammalia. The term "mammal" includes the sub-classes of humans and companion animals. "Companion animals," as used herein, include dogs and cats of all ages (e.g., puppies or kittens, adults between 1 and 6 years of age, seniors between 7 and 10 years of age, and super-seniors 11 years of age or older), and other mammals of like nutritional needs to dogs and cats. For example, other domesticated animals of like nutritional needs to a cat may include minks and ferrets, who can survive indefinitely and healthily on a nutritional composition designed to meet the nutritional needs of cats. It will be appreciated by one of skill in the art that dogs and cats have nutritional needs which differ in key aspects. At a fundamental level, dogs are omnivores, whereas cats are obligate carnivores. Further, nutritional needs are not necessarily consistent with phylogenetic or other non-nutritional classifications.

As used herein, the term "vegetarian" refers to a product, including but not limited to foods and supplements, which are not made from or with the aid of products derived from animals that have died, have been slaughtered, or animals that die as a result of being eaten. Animals means farmed, wild or domestic animals, including, but not limited to, livestock poultry, game, fish, shellfish, crustacea, amphibians, tunicates, echinoderms, mollusks and insects. In one example, a product can be vegetarian if it includes dairy products and eggs.

As used herein, the term "vegan" refers to a product including, but not limited to foods and supplements, which are not made from or with the aid of animals or animal products (including products from living animals).

As used herein, "visually perceptible" means that a human viewer can visually discern the cracking with the unaided eye (excepting standard corrective lenses adapted to compensate for near-sightedness, farsightedness, or stigmatism, or other corrected vision) in lighting at least equal to the illumination of a standard 100 watt incandescent white light bulb at a distance of about eighteen inches (about 45.72 cm).

As used herein, the articles "a" and "an" are understood to mean one or more of the material that is claimed or described, for example, "an active ingredient" or "a supplement".

It was surprisingly found that SCEs of only certain herbs caused vegetarian, non-GMO capsule shells to crack.

In one example, 320 mg turmeric (rhizome) hydroethanolic extract and 80 mg turmeric (rhizome) supercritical extract, extra virgin olive oil, maltodextrin, and yellow beeswax are put in a hydroxypropyl methylcellulose (HPMC) capsule shell. The capsule shell was size 00, with a volume of about 0.85 mL. After processing and over a year of storage in a warehouse at ambient conditions no cracking was observed.

However, in another example, 96 mg ginger (rhizome) hydroethanolic extract, 54 mg ginger (rhizome) supercritical extract and 5 mg rosemary (leaf) supercritical extract, extra virgin olive oil, maltodextrin, yellow beeswax, magnesium carbonate, and sunflower oil were put in an HPMC capsule shell. The capsule shell was size 2 capsule with a volume of about 0.34 mL. Within a few days of processing, during routine online inspection, an unacceptable level, approximately 2%, of the capsules appeared cracked.

To determine whether or not there is cracking, the following Cracking Test was used. The filled capsules are stored at ambient temperature. They were visually inspected to see if cracking was visually perceptible. The capsules were visually inspected for cracking immediately after sealing, at three days, five days, seven days, and ten days. If no cracking was visually perceptible after ten days, then the capsule passes. If cracking was visually perceptible before or at ten days, then the shell is cracked and it fails. In one example fewer than about 3% of capsules can break to pass the Cracking Test, in another example fewer than about 2%, and in another example fewer than about 0.2%, in another example fewer than about 0.02%, in another example fewer than about 0.002%. In one example, at least about 95% of capsules pass the Cracking Test, in another example at least about 97%, in another example at least about 98%, in another example at least about 99%, in another example at least about 99.5%, in another example at least about 99.8%, in another example at least about 99.9%, in another example at least about 99.95%, and in another example at least about 99.98%.

It was especially surprising that the capsules with the ginger extracts were cracked and the capsules with the turmeric extracts were not cracked and required no modification. While not being bound by theory, it is believed that the chemical makeup of certain SCEs causes the vegetarian, non-GMO capsules to split.

In one example, cracking was observed in capsules with ginger extracts and turmeric extracts.

Addressing this problem can require altering the composition of the product, including the composition of the fill. The concentration of supercritical ginger extract is reduced to less than about 10% of the fill phase. This required using a slightly larger capsule and in this case, a size 0 capsule, with a volume of about 0.61 mL, was used. First, the concentration of supercritical ginger extract was adjusted. The original idea was to adjust the concentration by adding more olive oil and beeswax, however, no more than 5% beeswax, based on the weight of the capsule, could be added in order to meet the non-GMO Project Standards. If only olive oil and beeswax were used, too much olive oil would need to be added in order to properly dilute the SCEs to the desired concentration and this would make the viscosity of the fill too thin. If the viscosity of the fill is too thin, it will be difficult to get the capsule to seal properly. Therefore, it was important to find another component, in addition to olive oil and beeswax, that would allow the fill to have the proper SCE concentration, correct viscosity, while meeting the non-GMO Project Standards. According to the present invention, the capsule fill contains candelilla wax in addition to beeswax.

According to the present invention, fewer capsule shells were cracked if a capsule shell was used that contained both gellan gum and HPMC, instead of solely HPMC.

According to the present invention, the capsule is vegetarian and in an example, the capsule can be vegan. In one example, the capsule can be dairy-free and in another example the capsule can be free of eggs and egg products. In another example the capsule can be kosher certified and in another example the capsule can be halal certified. In another example, all or some of the capsule ingredients can be organic. In another example, the capsule can be gluten free. In another example the capsule can be Bovine spongiform encephalopathy (BSE) free.

In one example, the capsule, including the capsule shell and fill phase, are substantially free of added colors and flavors.

According to the present invention the capsule comprises a volume greater than 0.61 mL. In an example, the capsule has a volume of less than about 1.36 mL, in another example less than about 1 mL, in another example less than about 0.92 mL, in another example less than about 0.85 mL, and in another example less than about 0.7 mL.

The capsule shell can be two-pieces, a body and a cap, which can be hermetically sealed to form a seal that is substantially leak-proof. After sealing, the capsule is one-piece and may not be opened without visible damage. The capsule shell can provide protection against tampering and leakage, and for actives that are moisture sensitive.

The capsule shell can be a hard shell or a soft shell. In one example, the capsule shell can be a hard shell.

In one example, the capsule shell can be substantially transparent. In another example, the capsule shell is colorless and/or substantially colorless. In one example, the capsule shell can be substantially free of artificial colors and/or flavors. In one example, the capsule shell can be colored with natural colorants, for instance carrots or beets.

In one example, the capsule shell can contain one or more polymers. According to the present invention, the polymers can be vegetarian and non-GMO. In one example, the capsule shell can contain a polymer selected from the group consisting of HPMC, pullulan, and combinations thereof. In one example, the capsule shell can be substantially free of gelatin. In another example, the capsule shell can be substantially free of gelling agents and/or other ingredients.

In another example, the capsule shell can contain fish gelatin, vegetable glycerin, sorbitol, and/or carob. The capsule shell can also contain coloring agents, preservatives, disintegrants, lubricants and surface treatments.

In one example, the fill phase can be a liquid and in another example the fill phase can be a semi-solid.

The fill phase can contain a SCE with a high percentage of essential oil. In one example, the fill phase can contain less than about 50% SCE, in another example less than about 40%, in another example less than about 33%, in another example less than about 20%, in another example less than about 15%, in another example less than about 12%, in another example less than about 11%, in another example less than about 10%, in another example less than about 8%, and in another example less than about 5%. In another example, the fill phase can contain at least about 3% SCEs, in another example at least about 5%, and in another example at least about 7%.

According to the present invention, the fill phase can includes beeswax and candelilla wax. Non-limiting examples of waxes can include beeswax, candelilla wax, carnauba wax, spermaceti, baysberry, montan, ozokerite, ceresin, paraffin, hydrogenated castor oil (castor wax), synthetic waxes such as Fisher-Tropsch waxes, microcrystalline wax and combinations thereof. Accoding to the present invention, the wax includes a combination of candelilla wax and beeswax.

In one example, the fill phase can contain from about 2% to about 20% wax, in another example from about 3% to about 15%, in another example from about 5% to about 13%, in another example from about 7% to about 11%, in another example from about 8% to about 10%, and in another example from about 8.5% to about 9.5%.

According to the present invention, the fill phase contains greater than 0% and less than 5% beeswax. In an example, the fill phase contains less than about 4% beeswax.

In another example, the fill phase can contain from about 1% to about 15% candelilla wax, in another example from about 2% to about 10%, in another example from about 2.3% to about 7%, in another example from about 2.6% to about 5%, in another example from about 2.8% to about 4.5%, and in another example from about 3% to about 3.6%.

In one example, the weight ratio of olive oil to wax in the fill phase is from about 2:1 to about 20:1, in another example from about 3:1 to about 15:1, in another example from about 4:1 to about 12:1, in another example from about 5:1 to about 9:1, and in another example from about 6:1 to about 8:1.

All such weights and percentages as they pertain to the waxes are based on the weight % of wax and do not include carriers or by-products that may be included in commercially available materials, unless otherwise specified.

In one example, the fill phase can contain beeswax, which has a melting point of 61-65°C. In one example, the fill phase can include at least one additional wax. The melting point of the additional wax can be from 55-86°C, in another example from about 60-80°C, in another example from about 65-75°C, and in another example from about 68-73°C. In one example, the additional wax can be candelilla wax, which has a melting point from 68-73°C.

According to the present invention, the fill phase contains both beeswax and candelilla wax. In one example, the weight ratio of beeswax to candelilla wax can be from about 1:3 to about 10:1, in another example from about 1:2 to about 5:1, in another example from about 2:3 to about 4:1, in another example from about 3:4 to about 3:1, in another example from about 1:1 to about 2:1, in another example from about 5:4 to about 7:4, and in another example from about 3:2 to about 5:3.

In one example, the fill phase can be substantially free of lecithin.

In another example, the fill phase can contain lecithin. In another example, the lecithin can be naturally sourced. Non-limiting examples of sources for lecithin can include soybeans, rapeseed, cottonseed, sunflowers, and combinations thereof. The lecithin is not sourced from eggs and/or milk and/or marine sources. In one example, the fill phase can contain from about 5% to about 75% lecithin, in another example from about 15% to about 70%, in another example from about 25% to about 70%, in another example from about 40% to about 60%, and in another example from about 45% to about 55%.

According to the present invention, the fill phase includes a supercritical extract of an herb comprising a supercritical extract of ginger and the supercritical extract of ginger is less than 10% of the fill phase. Non-limiting examples of herbals can include aloe vera, barberry, bilberry, black cohosh, black currant, calendula, cat's claw, caraway, cardamom, chaste tree (berry), Chinese goldthread, Chinese Skullcap, chamomile, cinnamon, clove, cranberry, dandelion, Echinacea, evening primrose, fennel, feverfew, flaxseed, garlic, ginger, gingko, ginseng, goldenseal, green tea, hawthorn, holy basil, horse chestnut, hu zhang, kava, lemon balm, licorice root, milk thistle, mistletoe, myrrh, oregano, parsley, peppermint, pumpkin seed oil, red clover, rosemary, saw palmetto, schizandra, sea buckthorn, St. John's wort, stinging nettle, thyme, turmeric, valerian, and combinations thereof.

In another example, the fill phase can include essential components of an herbal, as might be obtained by synthetic chemistry, isolation or purification from an SCE of the herbal, or alternate means of isolation or purification of the essential component. Non-limiting examples of essential components include gingerols, cinnamaldehyde, tumerones, carvacrol, and combinations thereof.

In one example, the fill phase can include SCEs, where supercritical CO₂ or traditional extraction techniques, which can deliver a concentrated herb, similar in composition to the whole herb. In one example, the supplement can deliver the wisdom of whole food. In another example, the extracts can provide the fullest spectrum of beneficial phytonutrients in their natural profile.

According to the present invention, the fill phase contains ginger. In one example, the fill phase can contain 96 mg ginger (rhizome) hydroethanolic extract and 54 mg ginger (rhizome) supercritical extract and/or 5 mg rosemary (leaf) supercritical extract. The capsule can support blood platelet health and cardiovascular function and can support the growth of beneficial intestinal microorganisms, which are vital for intestinal health. The capsule can also help with a healthy inflammation response.

In another example, the fill phase can contain cinnamon. In one example, the fill phase can contain 94 mg Cinnamon (*Cinnamomum aromaticum*) and (*Cinnamomum verum*) (bark) hydroethanolic extract and/or 46 mg Cinnamon (*Cinnamomum aromaticum*) and (*Cinnamomum verum*) (bark) supercritical extract. The capsule can provide healthy weight management by helping glucose create immediate cellular energy instead of stored potential energy in the form of fat deposits.

In another example, the fill phase can contain garlic. In one example, the fill phase can contain 160 mg garlic (bulb) aqueous extract and/or 80 mg garlic (bulb) supercritical extract, and 8 mg ginger (rhizome) supercritical extract and/or 4 mg clove (bulb) supercritical extract and/or 3 mg oregano (leaf) supercritical extract. The capsule can provide protection against free-radical damage and support cardiovascular health.

In another example, the fill phase can contain oregano. In one example, the fill phase can contain 256 mg oregano *(Origanum vulgare)* (leaf) aqueous extract and/or 125 mg oregano *(Origanum vulgare)* (leaf) supercritical extract. The capsule can help reduce oxidative stress and can be a free-radical scavenger, which can support a healthy immune system. The capsule can help support normal respiratory and digestive function.

In another example, the fill phase can contain lemon balm. In one example, the fill phase can contain 200 mg lemon balm (*Melissa officinalis*) (leaf) hydroethanolic extract and/or 100 mg lemon balm (*Melissa officinalis*) (leaf) supercritical extract. The capsule can support alertness, focus, and a healthy mood.

In another example, the fill phase can be an herbal hormonal balance formulated for pre and post-menopausal women. The fill phase can contain 200 mg evening primrose oil (*Oenothera biennis*) (seed) supercritical extract and/or 80mg schizandra (*Schisandra chinensis*) (berry) hydroethanolic extract and/or 40mg schizandra (*Schisandra chinensis*) (berry) supercritical extract and 140 mg ginger (*Zingiber officinale*) (rhizome) containing 28 mg supercritical extract and 112 mg hydroethanolic extract and/or 95 mg sage aqueous extract and/or 20 mg chaste tree (*Vitex agnus-castus*) (berry) extract, and/or 10 mg rosemary (*Rosmarinus officinalis*) (leaf) supercritical extract and/or 5mg fennel supercritical extract. The capsule can promote optimal hormonal balance and wellness.

In another example, the fill phase can contain 200 mg evening primrose oil (*Oenothera biennis*) (seed) supercritical extract, 160 mg schizandra (*Schisandra chinensis*) (berry) extract, 50 mg ginger (*Zingiber officinale*) (rhizome) containing 27 mg supercritical extract and 23 mg hydroethanolic extract, 40 mg black cohosh (*Cimicifuga racemosa*) (root and rhizome) extract, 40 mg chaste tree (*Vitex agnus-castus*) (berry) extract, and 10 mg rosemary (*Rosmarinus officinalis*) (leaf) supercritical extract. The capsule can support hormonal balance.

In another example, the fill phase can help provide holistic prostate support. The fill phase can contain 50 µg selenium, 320 mg saw palmetto (berry) supercritical extract, 100 mg green tea (leaf) aqueous extract, 96 mg organic pumpkin seed oil (seed), 80 mg ginger (rhizome) containing 43.2 mg supercritical extract and 36.8 mg hydroethanolic extract, 50 mg stinging nettle (*Urtica dioica*) (root) hydroethanolic extract 10:1, 50 mg stinging nettle (*Urtica dioica*) (root) hydroethanolic extract 5:1, and/or 10 mg rosemary (leaf) supercritical extract. The capsule can help support normal urine flow and prostate health.

In another example, the fill phase can contain 50 µg selenium, 320 mg saw palmetto (*Serenoa repens*) (berry) supercritical extract, 100 mg organic green tea (*Camellia sinensis*) (leaf) extract, 100 mg chinese skullcap (*Scutellaria baicalensis*) (root) hydroethanolic extract, 90 mg organic pumpkin seed oil (*Cucurbita pepo*) (seed), 80 mg ginger (*Zingiber officinalis*) (rhizome) containing 43.2 mg organic supercritical extract and 36.8 mg hydroethanolic extract, 75 mg rosemary *(Rosmarinus officinalis)* (leaf) containing 50 mg supercritical extract and 25 mg hydroethanolic extract, 62.5 mg turmeric (*Curcuma longa*) (rhizome) containing 12.5 mg organic supercritical extract and 50 mg hydroethanolic extract, 50 mg nettle (*Urtica dioica*) (root) hydroethanolic extract 10:1, 50 mg nettle (*Urtica dioica*) (root) hydroethanolic extract 5:1, 50 mg holy basil *(Ocimum sanctum)* (leaf) hydroethanolic extract, 40 mg hu zhang (*Polygonum cuspidatum*) (root and rhizome) hydroethanolic extract, 20 mg chinese goldthread (*Coptis chinensis*) (root) aqueous extract, 20 mg barberry (*Berberis vulgaris*) (root) hydroethanolic extract, and 20 mg organic oregano (*Origanum vulgare*) (leaf) supercritical extract.

In another example, the fill phase can contain herbs that can optimize immunoactive botanical pathways and contain multiple immune-supportive constituents. The fill phase can contain 40 mg garlic (*Allium sativum*) (bulb) containing 30 mg hydroethanolic extract and 10 mg supercritical extract, 30 mg oil of oregano (*Origanum vulgare)* (leaf) supercritical extract, 30 mg echinacea (*Echinacea purpurea*) (leaf and flower), 30 mg elderberry (*Sambucus nigra*) (berry) extract, 30 mg goldenseal (*Hydrastis canadensis*) (root) extract, 30 mg andrographis (*Andrographis paniculata*) (leaf) extract, 30 mg green tea (*Camellia sinensis*) (leaf) extract, 30 mg astragalus (*Astragalus membranaceus*) (root) extract, 30 mg lemon balm (*Melissa officinalis*) (leaf) supercritical extract, 30 mg myrrh (*Commiphora molmol*) (gum resin) containing 24 mg hydroethanolic extract and (*Commiphora abyssinica*) (gum resin) 6 mg supercritical extract, 15 mg wintergreen (*Gaultheria procumbens*) extract, 10 mg ginger (*Zingiber officinale*) (rhizome) containing 8 mg hydroethanolic extract and 2 mg supercritical extract, 7.5 mg eucalyptus (*Eucalyptus globulus*) (leaf) oil, 7.5 mg peppermint (Mentha × piperita) (leaf) supercritical extract, 7.5 mg meadowsweet (*Filipendula ulmaria*) extract, and/or 7.5 mg purple willow (*Salix purpurea*) extract. The capsule can support healthy sinus and respiratory function. The capsule can also help promote a healthy inflammation and antioxidant response that benefits more than just sinuses.

In another example, the fill phase can contain St. John's Wort. In one example, the fill phase can contain 1170 mg St. John's Wort hydroethanolic extract and supercritical extract. The capsule can support positive emotional health.

In another example, the fill phase can contain 500 mg of a blend of Sea Buckthorn (fruit and seed) supercritical extract, Rosemary supercritical extract, and/or Calendula supercritical extract.

In another example, the fill phase can provide pain relief after exercise. The fill phase can contain 150 mg rosemary *(Rosmarinus officinalis)* (leaf) containing 100 mg supercritical extract and 50 mg hydroethanolic extract, 110 mg turmeric (*Curcuma longa*) (rhizome) containing 10 mg organic supercritical extract and 100 mg hydroethanolic extract, 100 mg ginger (*Zingiber officinale*) (rhizome) containing 54 mg organic supercritical extract and 46 mg hydroethanolic extract, 100 mg holy basil (*Ocimum sanctum*) (leaf) hydroethanolic extract, 100 mg organic green tea (leaf), 80 mg hu zhang (*Polygonum cuspidatum*) (root and rhizome) hydroethanolic extract, 40 mg chinese goldthread (*Coptis chinensis*) (root) extract, 40 mg barberry (*Berberis vulgaris*) (root) hydroethanolic extract, 40 mg organic oregano (*Origanum vulgare*) (leaf) supercritical extract, and 20 mg chinese skullcap (*Scutellaria baicalensis*) (root) extract.

In another example, the fill phase can support sleep. The fill phase can contain 150 mg holy basil (*Ocimum sanctum*) (leaf) hydroethanolic extract, 100 mg turmeric (rhizome) containing 25 mg organic supercritical extract and 75 mg hydroethanolic extract, 100 mg chinese skullcap (*Scutellaria baicalensis*) (root) hydroethanolic extract, 85 mg lemon balm (*Melissa officinalis*) (leaf) supercritical extract, 75 mg chamomile (*Matricaria recutita*) (flower) containing 50 mg supercritical extract and 25 mg hydroethanolic extract, 75 mg hops (*Humulus lupulus*) (strobiles) containing 50 mg supercritical extract and 25 mg hydroethanolic extract, 75 mg ginger (*Zingiber officinalis*) (rhizome) containing 13.5 mg organic supercritical extract and 61.5 mg hydroethanolic extract, and 40 mg valerian (*Valeriana officinalis* and *Valeriana mexicanus*) (root) supercritical extract.

In another example, the fill phase can contain holy basil. In one example, the fill phase can contain 536 mg holy basil *(Ocimum sanctum)* (leaf) hydroethanolic extract and 536 mg holy basil *(Ocimum sanctum)* (leaf) supercritical extract. The capsule can help promote calm and balance.

In another example, the fill phase can contain 380 mg turmeric (*Curcuma longa*) (rhizome) containing 320 mg hydroethanolic extract and 60 mg supercritical extract, 300 mg organic green tea (*Camellia sinensis*) (leaf) extract, 15 mg ginger (*Zingiber officinale*) (rhizome) containing 12.2 mg hydroethanolic extract and 2.8 mg organic supercritical extract, 15 mg parsley (*Petroselinum crispum*) (leaf) containing 10 mg hydroethanolic extract and (seed) 5 mg supercritical extract, 15 mg rosemary (*Rosmarinus officinalis*) (leaf) containing 10 mg hydroethanolic extract and 5 mg supercritical extract, 10 mg clove (*Syzygium aromaticum*) (bud) containing 5 mg supercritical extract and 5 mg aqueous extract, and 5 mg peppermint (Mentha × piperita) (leaf) supercritical extract.

The fill phase can also contain additional ingredients. Non-limiting examples of additional ingredients can include olive oil, in particular extra virgin olive oil, maltodextrin, sunflower oil, silica, fermented soy, fermented yeast, gum acacia, and combinations thereof.

In one example, the capsule can have a shelf life of about 1 year or longer, in another example of about 18 months or longer, in another example of about 2 years or longer, in another example about 30 months or longer, and in another example of about 3 years or longer. The shelf life can be determined with stability studies.

A user can take one, two, three or more capsules daily. The capsules can be taken with or without food. The capsules can be taken in the morning, mid-day, the evening and/or at bedtime.

While the specification concludes with the claims particularly pointing and distinctly claiming the invention, it is believed that embodiments of the present invention will be better understood from this description. In all embodiments of the present invention, all weight percentages are by weight of the total composition, unless specifically stated otherwise. All ratios are weight ratios, unless specifically stated otherwise. All ranges are inclusive and combinable. The number of significant digits conveys neither limitation on the indicated amounts nor on the accuracy of the measurements. All measurements are understood to be made at 25°C and at ambient conditions, where "ambient conditions" means conditions under about one atmosphere of pressure and at about 50% relative humidity.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A capsule comprising:
a. a shell containg gellan gum and HPMC;
b. a fill phase comprising beeswax, candelilla wax, and a supercritical extract of an herb;
wherein the capsule is vegetarian and meets the non-GMO Project Standards;
wherein the capsule comprises greater than 0% and less than 5%, by weight of the capsule, beeswax; and
wherein at least 98% of the capsules pass the Cracking Test,
wherein the supercritical extract of an herb comprises a supercritical extract of ginger and the supercritical extract of ginger is less than 10% of the fill phase, and wherein the capsule comprises a volume greater than 0.61 mL.

2. The capsule according to claim 1 wherein the weight ratio of beeswax to candelilla wax is from 1:3 to 10:1, more preferred from 1:3 to about 5: 1, and most preferred from 1:1 to 2:1.

3. The capsule according to any one of the preceding claims wherein the fill phase comprises from 1% to about 15%, more preferred from 2% to 10%, and most preferred from 2.6% to 5% candelilla wax.

4. The capsule according to any one of the preceding claims further comprising an additional ingredient selected from the group consisting of extra-virgin olive oil, organic olive oil, organic sunflower oil, maltodextrin, and combinations thereof.

5. The capsule according to claim 4 wherein the additional ingredient is olive oil and the olive oil to wax ratio in the fill phase is from 4:1 to 12:1, more preferred from 5:1 to 9:1, and most preferred from 6:1 to 8: 1.

## Patentansprüche

1. Kapsel, umfassend:
a. eine Schale, die Gellangummi und HPMC enthält;
b. eine Füllphase, die Bienenwachs, Candelillawachs und einen überkritischen Extrakt eines Krauts umfasst;
wobei die Kapsel vegetarisch ist und die GVO-frei-Projekt-Standards erfüllt;
wobei die Kapsel zu mehr als 0 Gew.-% und weniger als 5 Gew.-% der Kapsel Bienenwachs umfasst; und
wobei mindestens 98 % der Kapseln die Rissprüfung bestehen,
wobei der überkritische Extrakt eines Krauts einen überkritischen Ingwerextrakt umfasst und der überkritische Ingwerextrakt weniger als 10 % der Füllphase beträgt, und wobei die Kapsel ein Volumen von mehr als 0,61 ml umfasst.

2. Kapsel nach Anspruch 1, wobei das Gewichtsverhältnis von Bienenwachs zu Candelillawachs von 1:3 bis 10:1, mehr bevorzugt von 1:3 bis 5:1 und am meisten bevorzugt von 1:1 bis 2:1 beträgt.

3. Kapsel nach einem der vorstehenden Ansprüche, wobei die Füllphase von 1 % bis etwa 15 %, mehr bevorzugt von 2 % bis 10 % und am meisten bevorzugt von 2,6 % bis 5 % Candelillawachs umfasst.

4. Kapsel nach einem der vorstehenden Ansprüche, ferner umfassend einen zusätzlichen Bestandteil, ausgewählt aus der Gruppe bestehend aus nativem Olivenöl Extra, Bio-Olivenöl, Bio-Sonnenblumenöl, Maltodextrin und Kombinationen davon.

5. Kapsel nach Anspruch 4, wobei der zusätzliche Bestandteil Olivenöl ist und das Verhältnis von Olivenöl zu Wachs in der Füllphase von 4:1 bis 12:1, mehr bevorzugt von 5:1 bis 9:1 und am meisten bevorzugt von 6:1 bis 8:1 beträgt.

## Revendications

1. Capsule comprenant :
a. une enveloppe contenant de la gomme gellane et de la HPMC ;
b. une phase de remplissage comprenant de la cire d'abeille, de la cire de candelilla et un extrait surcritique d'une plante ;
dans laquelle la capsule est d'origine végétale et répond aux normes du Projet sans OGM ;
dans laquelle la capsule comprend plus de 0 % et moins de 5 %, en poids de la capsule, de cire d'abeille ; et
dans laquelle au moins 98 % des capsules réussissent le test de fissuration,
dans laquelle l'extrait surcritique d'une plante comprend un extrait surcritique de gingembre et l'extrait surcritique de gingembre représente moins de 10 % de la phase de remplissage, et dans laquelle la capsule comprend un volume supérieur à 0,61 mL.

2. Capsule selon la revendication 1 dans laquelle le rapport pondéral de la cire d'abeille à la cire de candelilla va de 1:3 à 10:1, plus préférablement de 1:3 à environ 5:1, et le plus préférablement de 1:1 à 2:1.

3. Capsule selon l'une quelconque des revendications précédentes dans laquelle la phase de remplissage comprend de 1 % à environ 15 %, plus préférablement de 2 % à 10 %, et le plus préférablement de 2,6 % à 5 % de cire de candelilla.

4. Capsule selon l'une quelconque des revendications précédentes comprenant en outre un ingrédient supplémentaire choisi dans le groupe constitué d'huile d'olive extra-vierge, huile d'olive organique, huile de tournesol organique, maltodextrine et des combinaisons de celles-ci.

5. Capsule selon la revendication 4 dans laquelle l'ingrédient supplémentaire est de l'huile d'olive et le rapport de l'huile d'olive à la cire dans la phase de remplissage va de 4:1 à 12:1, plus préférablement de 5:1 à 9:1, et le plus préférablement de 6:1 à 8:1.
